# EUROPEAN PATENT APPLICATION

(11) **EP 4 438 610 A1**
(43) Date of publication of application: **02.10.2024**
(21) Application number: 22895272.7
(22) Date of filing: 11.10.2022
(51) Int. Cl.: C07F 9/10, A61K 31/661, A61P 25/00, A61P 29/00

(54) **COMPOUND, RACEMATE OF SAID COMPOUND, SALT OF SAID COMPOUND OR SAID RACEMATE, COMPOSITION, ANTI-INFLAMMATORY AGENT, THERAPEUTIC AGENT FOR DEMENTIA, AND THERAPEUTIC AGENT FOR RETT SYNDROME**

(30) Priority: 22.11.2021 JP 2021189168
(71) Applicant: Institute of Rheological Function of Food Co., Ltd., Fukuoka 811-2501 (JP); Kyushu University, National University Corporation, Nishi-ku Fukuoka-shi Fukuoka 819-0395 (JP)
(72) Inventor: FUJINO, Takehiko, Fukuoka-shi, Fukuoka 813-0043 (JP); MAWATARI, Shiro, Fukuoka-shi, Fukuoka 813-0016 (JP); OKAUCHI, Tatsuo, Kitakyushu-shi, Fukuoka 804-8550 (JP); NIWASE, Shamim, Fukuoka-shi, Fukuoka 810-0054 (JP); HONSHO, Masanori, Fukuoka-shi, Fukuoka 819-0395 (JP); NAKASHIMA, Kinichi, Fukuoka-shi, Fukuoka 819-0395 (JP); NAKASHIMA, Hideyuki, Fukuoka-shi, Fukuoka 819-0395 (JP)
(74) Representative: V.O.
(86) International application number: PCT/JP2022/037816
(87) International publication number: WO 2023/089986

(57) **Abstract**

A compound is represented by Formula (I), 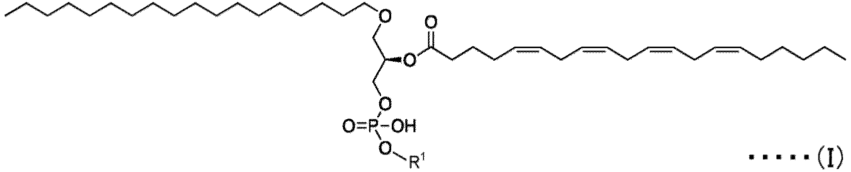 in which each carbon atom of a glycerol backbone optionally has one or more substituents, and R¹ is 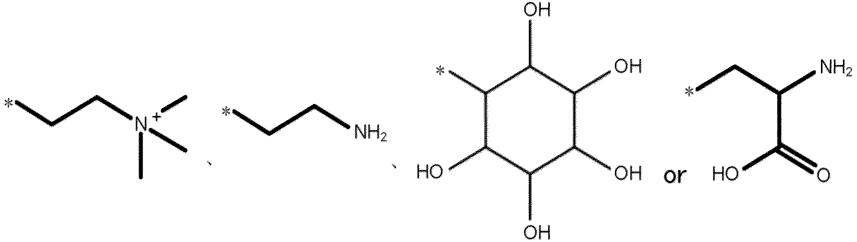 optionally having one or more substituents, in which * is an oxygen atom bound to R¹.

## Description

### Technical Field

The present disclosure relates to a compound, a racemate of the compound, a salt of the compound or the racemate, a composition, an anti-inflammatory agent, a dementia therapeutic agent, and a Rett syndrome therapeutic agent.

### Background Art

Plasmalogens are a type of phospholipids having antioxidant action, and are glycerophospholipids. Plasmalogens are present in all mammalian tissues, and account for about 18% of phospholipids in the human body. In particular, plasmalogens are known to be present in large amounts in cranial nerves, heart muscle, skeletal muscle, leukocytes, and spermatozoa.

Since most plasmalogens are bound to polyunsaturated fatty acids such as docosahexaenoic acid and arachidonic acid, they are involved in the storage of polyunsaturated fatty acids and the release of secondary messengers of intercellular signals such as prostaglandins and leukotriene produced from the polyunsaturated fatty acids. Plasmalogens are also involved in, for example, cell fusion and ion transport. Since the vinyl ether bond (alkenyl bond) of plasmalogens is particularly sensitive to oxidative stress, plasmalogens also have antioxidant function for cells.

In addition, plasmalogens are known to have an action to promote neurogenesis, an action to suppress neuroinflammation due to lipopolysaccharide (LPS), and an action to suppress accumulation of amyloid β (Aβ) protein in the brain. For example, Patent Literature 1 discloses a drug against central nervous system inflammation containing a plasmalogen.

Plasmalogens are known to be decreased in cranial nerve diseases such as dementia, Parkinson's disease, depression, and schizophrenia; and also in diabetes, metabolic syndrome, ischemic heart disease, infections, and immunopathy. For example, Non Patent Literature 1 reports that ethanolamine-type plasmalogen is highly significantly decreased in the frontal lobe and hippocampus in the brain in Alzheimer's disease. Non Patent Literature 2 reports that plasmalogen is decreased in sera of patients with Alzheimer's disease. Non Patent Literature 3 reports that choline-type plasmalogen is decreased in a group of patients with ischemic heart disease relative to a normal control group.

An effect for the prevention and amelioration of the diseases can be expected by external supplementation of the plasmalogen that has been decreased. Therefore, attempts have been made to extract plasmalogens from animal tissues. For example, Patent Literature 2 proposes a method in which breast meat of a layer is subjected to extraction treatment using ethanol as an extraction solvent, and then the resulting extract is collected.

Patent Literature 3 proposes a method including: subjecting a bivalve such as a scallop to extraction treatment using a mixed solvent of a nonpolar organic solvent and a branched alcohol; and treating the resulting extract with phospholipase A1 (PLA1) to remove diacyl-type glycerophospholipid as an impurity therefrom by hydrolysis. Non Patent Literature 4 reports that a randomized double-blind clinical trial was carried out by oral administration of a plasmalogen extracted from scallop mantle to human patients with mild Alzheimer's disease or mild dementia, and that the result of the trial strongly suggested improvement in the cognitive function in the mild Alzheimer's disease.

On the other hand, for the purpose of preventing or improving various diseases caused by deficiency of plasmalogens, synthesis of plasmalogen derivatives has been attempted to increase the plasmalogen level that has decreased. Patent Literature 4 discloses novel plasmalogen precursors in which α-lipoic acid is bound to the sn-3 position of a plasmalogen. Non Patent Literature 5 describes that the derivatives are effective in a monkey model of Parkinson's disease.

Patent Literature 5 proposes that a derivative in which the sn-1 position is acetylated is a good carrier for docosahexaenoic acid. Non Patent Literature 6 reports that the derivative is effective in a rat model of acute stroke.

### Citation List

### Patent Literature

Patent Literature 1: International Publication No. WO 2012/039472
Patent Literature 2: Unexamined Japanese Patent Application Publication No. 2010-063406
Patent Literature 3: Unexamined Japanese Patent Application Publication No. 2016-108466
Patent Literature 4: International Publication No. WO 2010/071988
Patent Literature 5: International Publication No. WO 2013/037862

### Non Patent Literature

Non Patent Literature 1: Z. Guan and others, Decrease and Structural Modifications of Phosphatidylethanolamine Plasmalogen in the Brain with Alzheimer Disease, J Neuropathol Exp Neurol, 58 (7), 740-747, 1999
Non Patent Literature 2: D. B. Goodenowe and others, Peripheral ethanolamine plasmalogen deficiency: a logical causative factor in Alzheimer's disease and dementia, J Lipid Res, 48, 2485-2498, 2007
Non Patent Literature 3: Takeo Sanada and others, Serum Plasmalogens in Ischemic Heart Disease, The Journal of Japan Atherosclerosis Society, 11, 535-539, 1983
Non Patent Literature 4: T. Fujino and others, Efficacy and Blood Plasmalogen Changes by Oral Administration of Plasmalogen in Patients with Mild Alzheimer's Disease and Mild Cognitive Impairment: A Multi center, Randomized, Double-blind, Placebo-controlled Trial, EBioMedicine, 17: 199-205, 2017
Non Patent Literature 5: L. Gregoire and others, Plasmalogen precursor analog treatment reduces levodopa-induced dyskinesias in parkinsonian monkeys, Behav Brain Res, 286: 328-337, 2015
Non Patent Literature 6: C. Fabien and others, Brain-Targeting Form of Docosahexaenoic Acid for Experimental Stroke Treatment: MRI Evaluation and AntiOxidant Impact, Current Neurovascular Res, 8 (2): 95-102, 2011

### Summary of Invention

### Technical Problem

An obj ective of the present disclosure is to provide a compound, a racemate of the compound, a salt of the compound or the racemate, a composition, an anti-inflammatory agent, a dementia therapeutic agent for dementia, and a Rett syndrome therapeutic agent for Rett syndrome, that show excellent anti-inflammatory action.

### Solution to Problem

The present inventors carried out research on plasmalogens and analogs thereof, to discover that production of an excellent anti-inflammatory action, and improvement of Rett syndrome-like symptoms in model animals of Rett syndrome, are possible even without the vinyl ether bond at the sn-1 position, that is a major characteristic of plasmalogens, thereby completing the present disclosure.

A compound, a racemate of the compound, or a salt of the compound or the racemate according to a first aspect of the present disclosure are a compound of Formula (I), a racemate of the compound, or a salt of the compound or the racemate, 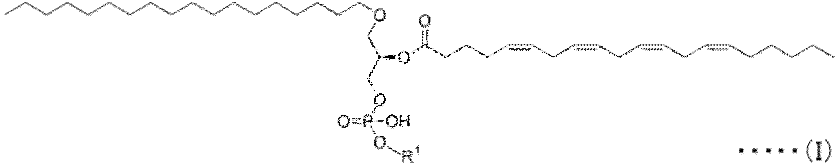 in which each carbon atom of a glycerol backbone optionally has one or more substituents, and R¹ is 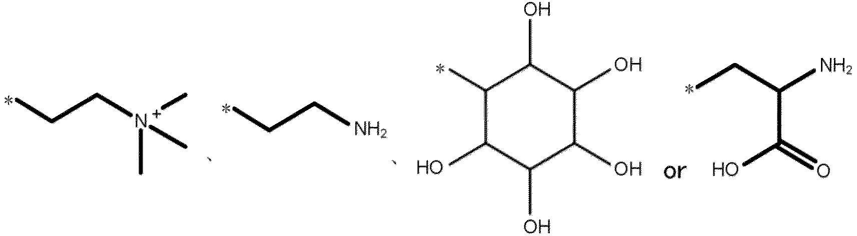 optionally having one or more substituents, in which * is an oxygen atom bound to R¹.

A composition according to a second aspect of the present disclosure includes the compound, the racemate of the compound, or the salt of the compound or the racemate according to the first aspect of the present disclosure, as an effective component.

An anti-inflammatory agent according to a third aspect of the present disclosure includes the composition according to the second aspect of the present disclosure.

The anti-inflammatory agent according to the third aspect of the present disclosure may be for prevention or improvement of a cranial nerve inflammatory disease.

A dementia therapeutic agent according to a fourth aspect of the present disclosure includes the composition according to the second aspect of the present disclosure.

A Rett syndrome therapeutic agent according to a fifth aspect of the present disclosure includes the composition according to the second aspect of the present disclosure.

### Advantageous Effects of Invention

The compound or the like according to the present disclosure exhibits an excellent anti-inflammatory action. In addition, the compound or the like according to the present disclosure improves symptoms of dementia and Rett syndrome.

### Brief Description of Drawings

FIG. 1 is a diagram illustrating the expression level of nuclear p65 induced by the LPS according to Test Example 1;
FIG. 2 is a diagram illustrating the expression level of IL-1β induced by the LPS according to Test Example 1;
FIG. 3 is a diagram illustrating the expression level of IL-1β induced by the LPS according to Test Example 2;
FIG. 4 is a diagram illustrating the number of days of survival of mice according to Test Example 3;
FIG. 5 is a diagram illustrating changes in the Rett syndrome (hereinafter also referred to as "RTT")-like score over time according to Test Example 4;
FIG. 6 is a diagram illustrating the body weights of mice according to Test Example 5; and
FIG. 7 is a diagram illustrating the result of a water maze test for model mice of LPS-induced memory impairment according to Test Example 6.

### Description of Embodiments

Embodiments according to the present disclosure are described below with reference to drawings. The present disclosure is not limited by the following embodiments and drawings. In the following embodiments, expressions such as "have", "include", or "contain" also encompass the meaning of "consist of" or "be constituted by".

A compound according to the present embodiment is represented by Formula (I). Each carbon atom of the glycerol backbone in Formula (I) may have one or more substituents. Examples of the substituents of the carbon atom of the glycerol backbone include hydroxy, halogen, aryl, C₁-C₄ alkyl, and C₁-C₄ alkoxy.

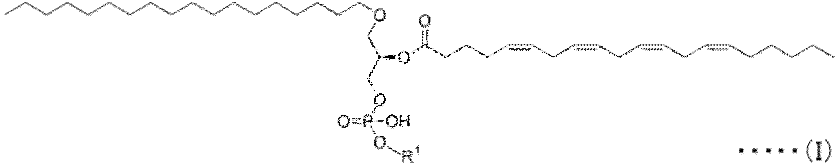

In Formula (I), R¹ is 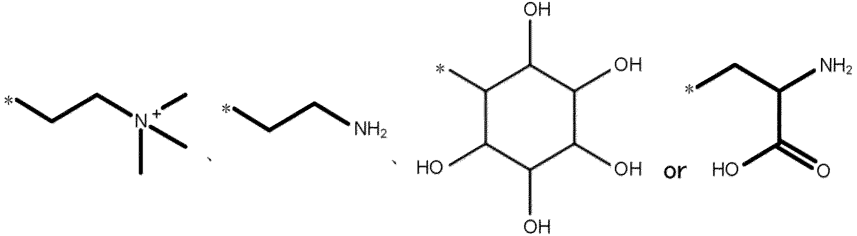 optionally having one or more substituents, in which * is an oxygen atom bound to R¹. Examples of the substituents of R¹ include hydroxy, halogen, aryl, C₁-C₄ alkyl, and C₁-C₄ alkoxy.

The compound according to the present embodiment is preferably represented by the following Formula (II).

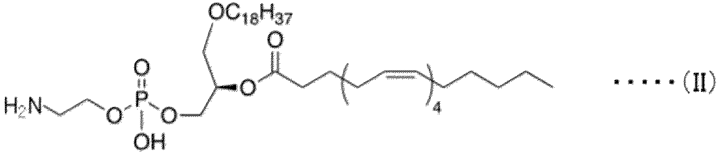

In the present embodiment, a racemate of the compound, a salt of the compound, and a salt of the racemate are provided. The salts are not limited as long as they are pharmacologically acceptable salts, and may be either acidic or basic salts. Examples of the salts include alkali metal salts such as lithium salt, sodium salt, and potassium salt; alkaline earth metal salts such as magnesium salt and calcium salt; inorganic acid salts such as hydrochloride, hydrobromide, sulfate, nitrate, oxalate, and phosphate; and organic acid salts such as acetate, propionate, hexanoate, cyclopentane propionate, glycolate, pyruvate, lactate, malonate, succinate, malate, fumarate, tartrate, citrate, benzoate, o-(4-hydroxybenzoyl)benzoate, cinnamate, mandelate, methanesulfonate, ethanesulfonate, 1,2-ethanedisulfonate, 2-hydroxyethanesulfonate, benzenesulfonate, p-chlorobenzenesulfonate, 2-naphthalenesulfonate, p-toluenesulfonate, camphorsulfonate, glucoheptanoate, 3-phenylpropionate, trimethylacetate, tertiary butyl acetate, lauryl sulfate, gluconate, glutamate, hydroxynaphthoate, salicylate, stearate, trifluoroacetate (TFA), maleate, and muconate.

The compound according to the present embodiment can be synthesized by a known method. For example, in cases where a compound containing ethanolamine as R¹ is to be synthesized, first, arachidonic acid is reacted with *tert-*butyl(2-((*tert-*butoxy((*R*)-2-hydroxy-3-(octadecyloxy)propoxy)phosphoryl)oxy)ethyl)carbamate using a condensing agent. The resulting product is deprotected by allowing a strong acid such as trifluoroacetic acid to act on the product, to obtain a compound according to the present embodiment. The condensing agent is not limited, and may be, for example, 1-(3-dimethylaminopropyl)-3-ethylcarbodiimide (EDC) hydrochloride, dicyclohexylcarbodiimide (DCC), HATU, HBTU, TATU, TBTU, and diphenylphosphoryl azide (DPPA). For promoting the dehydration condensation reaction by the condensing agent, a nucleophile such as dimethylaminopyridine (DMAP) may be used.

The compound according to the present embodiment has a novel structure, and has an anti-inflammatory action as shown by the Examples below.

Another embodiment provides a composition including: a compound represented by Formula (I), a racemate of the compound, or a salt of the compound or the racemate (hereinafter also referred to as "compound or the like"), as an effective component. The composition may also contain another pharmacologically acceptable component.

As shown in Examples below, the composition has an anti-inflammatory action. Therefore, the composition can be used for the prevention or treatment of an inflammatory disease, as an anti-inflammatory agent. The composition is particularly effective in the prevention or treatment of a cranial nerve inflammatory disease. Examples of the cranial nerve inflammatory disease include dementia, Parkinson's disease, depression, and schizophrenia. The composition is particularly effective for Alzheimer's dementia. Preferably, a dementia therapeutic agent, including the composition as an effective component is provided.

Further, as shown in Examples below, the composition is effective for the prevention or treatment of RTT that is a progressive neurodevelopmental disorder caused by mutation of the MeCP2 gene that is located on the X chromosome. Therefore, the composition can be used as an effective component of a therapeutic agent for RTT. RTT occurs mainly in female infants. Patients with RTT show normal growth for about half a year to one year after birth, and then develop various neurological symptoms such as autistic tendency. Although the following description is given for an anti-inflammatory agent containing the above composition, the description is also applicable to a dementia therapeutic agent and a therapeutic agent for RTT by replacing the anti-inflammatory agent with the dementia therapeutic agent and the therapeutic agent for RTT, respectively.

The content of the compound or the like in the anti-inflammatory agent is appropriately adjusted. For example, the anti-inflammatory agent includes the compound or the like at 0.00001 to 99.9% by mass, 0.0001 to 99.8% by mass, 0.001 to 99.7% by mass, 0.005 to 99.6% by mass, 0.01 to 99.5% by mass, 0.1 to 99% by mass, 0.5 to 60% by mass, 1 to 50% by mass, or 1 to 2% by mass as an effective component.

The subject for which the anti-inflammatory agent is to be used is a cell, a biological tissue, an organism, an animal, or the like. The anti-inflammatory agent as a pharmaceutical is administered to a human or an animal. Preferred examples of the animal include mammals, more specifically, dogs, cats, cows, pigs, horses, sheep, and deer.

The administration route of the anti-inflammatory agent for the human or the like is not limited. The anti-inflammatory agent is preferably used as an external preparation, an injection solution, or an orally administered agent. The anti-inflammatory agent may include, for example, the compound or the like and a pharmaceutically acceptable carrier. Examples of the pharmaceutically acceptable carrier include various organic carrier substances and inorganic carrier substances used as formulation materials. Specific examples of the pharmaceutically acceptable carrier include: excipients, lubricants, binders, and disintegrators in solid formulations; and solvents, solubilizers, suspending agents, isotonic agents, buffers, and soothing agents in liquid formulations. When necessary, additives such as antiseptics, antioxidants, coloring agents, and sweeteners may be added.

The anti-inflammatory agent is produced by a known method, and provided in the form of liquids, creams, ointments, tablets, granules, fine granules, powders, tablets, capsules, or the like.

The dose of the anti-inflammatory agent is appropriately determined in accordance with the age, body weight, symptoms, and the like of the human or animal to whom the agent is to be administered. The anti-inflammatory agent is administered to achieve an effective amount of the compound or the like. The effective amount is an amount of the compound or the like required for obtaining a desired result, and is an amount required for delaying, inhibiting, preventing, or reversing the progression of, or for curing of, the condition to be treated or handled.

The compound or the like according to the present embodiment may be used as a reagent in an in vitro or in vivo experiment.

In another embodiment, the above composition is provided as an anti-inflammatory oral composition, an oral composition for treatment of dementia, or an oral composition for treatment of RTT. Specific examples of the anti-inflammatory oral composition, the oral composition for treatment of dementia, or the oral composition for treatment of RTT include supplements, food compositions, foods and beverages, functional foods, and food additives.

The forms of the supplements are not limited, and may be any of tablets, powders, granules, capsules, sugar-coated tablets, film formulations, troches, chewable formulations, solutions, emulsions, and suspensions. The supplements may include any component that is normally used for supplements.

"Functional food" means a food or beverage consumed for the purpose of maintaining health, and examples of the functional food include: foods for specified health uses, foods with function claims, and foods with nutrient function claims, which are foods with health claims; health foods; and dietary supplements. The functional food is preferably a food for specified health uses or a food with nutrient function claims, that is a food with health claims. In cases of commercialization as a functional food, the anti-inflammatory oral composition may include various additives for use in foods, such as colorants, preservatives, thickening stabilizers, antioxidants, bleaching agents, antibacterial and antifungal agents, acidulants, sweeteners, seasonings, emulsifiers, fortifiers, agents for production, and flavors.

The functional food may be either a food or beverage, and is not limited as long as the functional food can be orally taken. Examples of the form of the functional food include beverages, confectionery, processed cereal products, kneaded products, dairy products, and seasonings. Examples of the beverages include nutritional drinks, soft drinks, black tea, and green tea. Examples of the confectionery include candies, cookies, tablets, chewing gums and jellies. Examples of the processed cereal product noodles include bread, cooked rice, and biscuits. Examples of the kneaded products include sausage, ham, and kamaboko (boiled fish paste). Examples of the dairy products include butter and yogurt.

The anti-inflammatory oral composition, the oral composition for treatment of dementia, and the oral composition for treatment of RTT may be added to a food as a food additive. In such a case, examples of the food additive may include pastes, gel-like agents, powders, liquids, suspensions, emulsions, and granules, from the viewpoint of easily adding the food additive to foods.

Each of the anti-inflammatory oral composition, the oral composition for treatment of dementia, and the oral composition for treatment of RTT may contain water, vitamins, minerals, organic acids, organic bases, fruit juices, flavors, functional components, food additives, and the like as long as the anti-inflammatory action or the RTT-improving action is maintained. The anti-inflammatory oral composition, the oral composition for treatment of dementia, and the oral composition for treatment of RTT may be produced by a known method in which components other than the compound or the like are added, when necessary.

The anti-inflammatory oral composition, the oral composition for treatment of dementia, and the oral composition for treatment of RTT may be divided into one or more containers such that the daily intake is in accordance with the above-described amount of intake. In such a case, the anti-inflammatory oral composition, the oral composition for treatment of dementia, or the oral composition for treatment of RTT is preferably contained in each container in an amount corresponding to the daily intake.

From the viewpoint of the fact that the anti-inflammatory oral composition, the oral composition for treatment of dementia, and the oral composition for treatment of RTT are used for an inflammatory disease, dementia, or RTT, they are provided in the forms of products that can be distinguished from other products. For example, at least one of the product packaging, instructions, and advertisements for the anti-inflammatory oral composition indicates that the composition has an anti-inflammatory action. At least one of the product packaging, instructions, and advertisements for the oral composition for treatment of dementia indicates that the composition has a dementia-improving action. At least one of the product packaging, instructions, and advertisements for the oral composition for treatment of RTT indicates that the composition has an RTT improving action.

### EXAMPLES

The present disclosure is described more concretely by way of the following Examples. However, the present disclosure is not limited by Examples.

### Example 1: Synthesis of Compound KIT-13

KIT-13 was synthesized as a compound according to Formula (I) as follows.

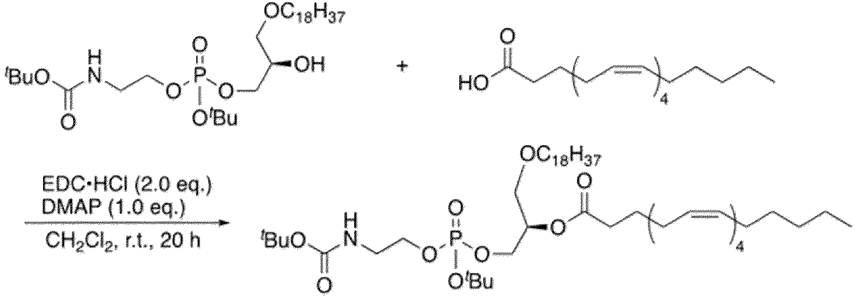

To a solution of tert-butyl(2-((tert-butoxy((R)-2-hydroxy-3-(octadecyloxy)propoxy)phosphoryl)oxy)ethyl)carbamate (187 mg, 0.30 mmol) in dichloromethane (3 mL), EDC hydrochloride (115 mg, 0.60 mmol), DMAP (37 mg, 0.30 mmol), and arachidonic acid (100 mg, 0.33 mmol) were added at room temperature, and the resulting mixture was stirred for 20 hours. The reaction solution was diluted with ethyl acetate, and the resulting dilution was washed with water. The ethyl acetate layer was dried over anhydrous sodium sulfate, and then concentrated under reduced pressure. The residue was purified by column chromatography, to obtain a desired product, (2R)-1-((tert-butoxy(2-((tert-butoxycarbonyl)amino)ethoxy)phosphoryl)oxy)-3-(octadecyloxy)propan-2-yl(5*Z*,8*Z*,11*Z*,14*Z)*-icosa-5,8,11,14-tetraenoate, as a colorless liquid (260 mg, 95%).

¹H NMR (500 MHz, CDCl₃) δ 5.43-5.31 (m, 8H), 5.16 (quin, J = 4.9 Hz, 1H), 5.16 (br, 1H), 4.21 (m, 1H), 4.14-4.09 (m, 1H), 4.07-4.02 (m, 2H), 3.55 (d, J = 5.3 Hz, 2H), 3.47-3.38 (m, 5.3 Hz), 2.85-2.79 (m, 6H), 2.35 (t, J = 7.6 Hz), 2.12 (q, J = 7.0 Hz, 2H), 2.06 (q, J = 7.1 Hz, 2H), 1.74-1.67 (m, 2H), 1.56-1.54 (m, 2H), 1.502 (s, 9H, one diastereomer), 1.496 (s, 9H, the other diastereomer), 1.39-1.25 (m, 38H), 0.90-0.87 (m, 6H).

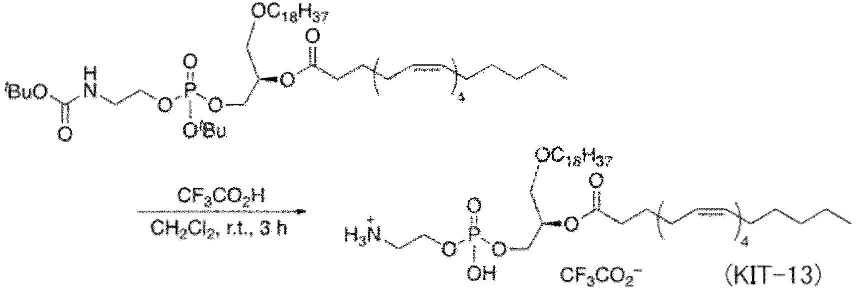

To a solution of (2*R*)-1-((*tert*-butoxy(2-((*tert-*butoxycarbonyl)amino)ethoxy)phosphoryl)oxy)-3-(octadecyloxy)propan-2-yl(5Z,8Z, 11Z, 14Z)-icosa-5,8, 11, 14-tetraenoate (260 mg, 0.29 mmol) in dichloromethane (2 mL), 2 mL of trifluoroacetic acid was added at room temperature, and the resulting mixture was stirred for 3 hours. The reaction mixture was concentrated under reduced pressure, to obtain a desired product, 2-(hydroxy((*R*)-2-(((5*Z*,8*Z*,11*Z*,14*Z*)-icosa-5,8,11,14-tetraenoyl)oxy)-3-(octadedecyloxy)propoxy)phosphoryl)oxy)ethane-1-ammonium 2,2,2-trifluoroacetate (KIT-13), as a colorless liquid (249 mg, quant.)

¹H NMR (500 MHz, CDCl₃) δ 11.31 (br, 1H), 5.41-5.31 (m, 8H), 5.16-5.15 (m, 1H), 4.17 (br, 2H), 4.07-4.00 (m, 2H), 3.54 (d, J = 4.9 Hz, 2H), 3.45-3.39 (m, 2H), 3.27 (br), 2.84-2.78 (m, 6H), 2.35 (t, J = 7.4 Hz, 2H), 2.10 (q. J = 7.1 Hz, 2H), 2.05 (q. J = 7.2 Hz, 2H), 1.69-1.66 (m, 2H), 1.54-1.51 (m, 2H), 1.39-1.25 (m, 38H), 0.90-0.86 (m, 6H).

KIT-2 was synthesized as follows.

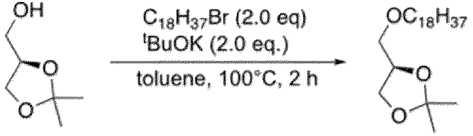

To a suspension of ^{t}BuOK (2.2 g, 20 mmol) in toluene (13 mL), a solution of (*R*)-(2,2-dimethyl-1,3-dioxolan-4-yl)methanol (1.3 g, 10 mmol) in toluene (10 mL) was added at 0°C, and the resulting mixture was stirred for 1 hour. To the mixture, a solution of 1-bromooctadecane in toluene (10 mL) was added. The resulting mixture was heated to 100°C, and then stirred for 2 hours. The mixture was cooled to 0°C, and then saturated aqueous ammonium chloride solution was added thereto, followed by performing two times of extraction with Et₂O. The Et₂O layers obtained by the extraction were combined. The resulting combined layer was washed with water and saturated brine, and dried over Na₂SO₄, followed by removal of the solvent by distillation under reduced pressure. The residue was purified by silica gel column chromatography, to obtain a desired product, (R)-2,2-dimethyl-4-((octadecyloxy)methyl)-1,3-dioxolane, as a white solid (3.7 g, 96%).

¹H NMR (500 MHz, CDCl₃) δ 4.28-4.23 (m, 1H), 4.06 (dd, J = 8.2, 6.4 Hz, 1H), 3.73 (dd, J = 8.3, 6.4 Hz, 1H), 3.53-3.41 (m, 4H), 1.59-1.54 (m, 2H), 1.42 (s, 3H), 1.36 (s, 3H), 1.25 (m, 30H), 0.88 (t, J = 7.0 Hz, 3H).

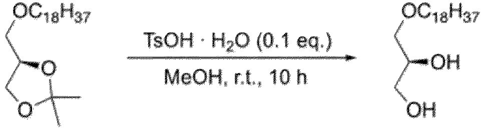

To a solution of (R)-2,2-dimethyl-4-((octadecyloxy)methyl)-1,3-dioxolane (3.89 g, 10.1 mmol) in methanol (40mL), tosic acid monohydrate (397 mg, 2.0 mmol) was added at room temperature. After stirring the resulting mixture for 10 hours, triethylamine (0.6 mL) was added thereto. The resulting mixture was concentrated under reduced pressure. The residue was purified by column chromatography, to obtain a desired product, (*S*)-3-(octadecyloxy)propan-1,2-diol, as a white solid (3.25 g, 93%).

¹H NMR (500 MHz, CDCl₃) δ 3.88-3.84 (m, 1H), 3.74-3.70 (m, 1H), 3.67-3.63 (m, 1H), 3.55-3.43 (m, 4H), 2.67 (d, J = 5.1 Hz, 1H), 2.25 (d, J = 7.0, 5.2 Hz, 1H), 1.59-1.55 (m, 2H), 1.26 (m, 30H), 0.88 (t, J = 6.9 Hz, 3H).

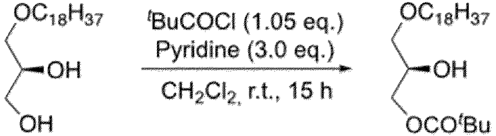

To a solution of (*S*)-3-(octadecyloxy)propan-1,2-diol (1.36 g, 4.0 mmol) and pyridine (1.0 mL, 12 mmol) in dichloromethane (40 mL), pivaloyl chloride (0.5 mL, 4.2 mmol) was added at room temperature, and the resulting mixture was stirred for 15 hours. Phosphate buffer (pH 7) was added to the mixture at 0°C, and extraction with ethyl acetate was carried out twice. The ethyl acetate layers obtained by the extraction were combined, and washed with water and saturated brine. The resulting combined layer was dried over Na₂SO₄, and then the solvent was removed by distillation under reduced pressure. The residue was purified by column chromatography, to obtain a desired product, (R)-2-hydroxy-3-(octadecyloxy)propyl pivalate, as a colorless liquid (1.28 g, 75%).

¹H NMR (500 MHz, CDCl₃) δ 4.18-4.11 (m, 2H), 4.02-3.67 (m, 1H), 3.51-3.41 (m, 4H), 2.47 (d, J = 4.8 Hz, 1H), 1.57 (quin, J = 6.8 Hz, 2H), 1.25 (m, 30H), 1.22 (s, 9H), 0.88 (t, J = 7.0 Hz, 3H).

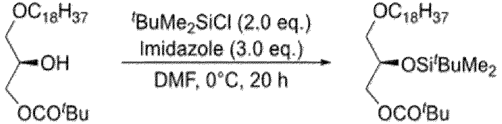

To a solution of (R)-2-hydroxy-3-(octadecyloxy)propyl pivalate (1.2 g, 2.8 mmol) and imidazole (570 mg, 8.4 mmol) in DMF (8 mL), *tert-*butyldimethylchlorosilane (840 mg, 5.6 mmol) was added at 0°C, and the resulting mixture was heated to room temperature, followed by stirring the mixture for 20 hours. Phosphate buffer (pH 7) was added to the mixture, and extraction with ethyl acetate was carried out twice. The ethyl acetate layers obtained by the extraction were combined. The resulting combined layer was washed with water and saturated brine, and dried over Na₂SO₄, followed by removal of the solvent by distillation under reduced pressure. The residue was purified by column chromatography, to obtain a desired product, (*R*)-2-((tertbutyldimethylsilyl)oxy)-3-(octadecyloxy)propyl pivalate, as a colorless liquid (1.5 g, 98%).

¹H NMR (500 MHz, CDCl₃) δ 4.17-4.13 (m, 1H), 4.00-3.95 (m, 2H), 3.42 (dt, J_{d} = 1.3 Hz, Jₜ = 6.7 Hz, 2H), 3.39 (d, J = 5.6 Hz, 2H), 1.54 (quin, J = 7.0 Hz, 2H), 0.89-0.86 (m, 12H), 0.087 (, 6H).

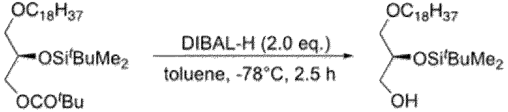

To a solution of (*R*)-2-((*tert*-butyldimethylsilyl)oxy)-3-(octadecyloxy)propyl pivalate (3.16 g, 5.82 mmol) in toluene (23 mL), a solution of 1 mol/L diisobutylaluminum hydride in toluene (11.6 mL) was added at -78°C, and the resulting mixture was stirred for 2.5 hours. To the mixture, 30% aqueous Rochelle salt solution was added, and the resulting mixture was stirred at 0°C for 30 minutes, followed by filtration through Cerite 545 and two times of extraction with ethyl acetate. The ethyl acetate layers obtained by the extraction were combined. The resulting combined layer was washed with water and saturated brine, and dried over Na₂SO₄, followed by removal of the solvent by distillation under reduced pressure. The residue was purified by column chromatography, to obtain a desired product, (*S*)-2-((*tert*-butyldimethylsilyl)oxy)-3-(octadecyloxy)propan-1-ol, as a colorless liquid (2.27 g, 85%).

¹H NMR (500 MHz, CDCl₃) δ 3.90-3.86 (m, 1H), 3.66-3.62 (m, 1H), 3.60-3.55 (m, 1H), 3.44-3.89 (m, 4H), 2.14 (dd, J = 7.5, 5.1 Hz, 1H), 1.55 (quin, J = 7.1 Hz, 2H), 1.25 (m, 30H), 0.90 (m, 12H), 0.099 (s, 3H), 0.096 (s, 3H).

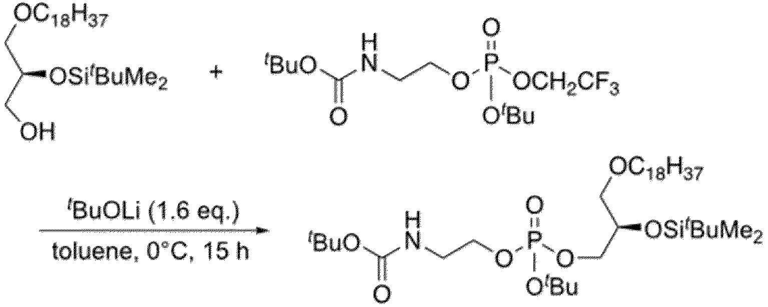

To a solution of (*S*)-2-((*tert-*butyldimethylsilyl)oxy)-3-(octadecyloxy)propan-1-ol (3.4 g, 7.4 mmol) in toluene (10 mL), a solution of 1.0 mol/L ^{t}BuOLi in hexane (12 mL, 12 mmol) was added at 0°C, and the resulting mixture was stirred for 1 hour. To the mixture, a solution of *tert*-butyl(2-((*tert*-butoxy(2,2,2-trifluoroethoxy)phosphoryl)oxy)ethyl)carbamate (2.81 g, 7.42 mmol) in toluene (10 mL) was added at 0°C, and the resulting mixture was stirred for 15 hours. Phosphate buffer (pH 7) was added to the mixture, and extraction with ethyl acetate was carried out twice. The resulting ethyl acetate layers were combined. The resulting combined layer was washed with water and saturated brine, and dried over Na₂SO₄, followed by removal of the solvent by distillation under reduced pressure. The residue was purified by column chromatography, to obtain a desired product, *tert-*butyl(2-((*tert*-butoxy((*R*)-2-((*tert*butyldimethylsilyl)oxy)-3-(octadecyloxy)propoxy)phosphoryl)oxy)ethyl)carbamate, as a colorless liquid (5.0 g, 91%).

¹H NMR (500 MHz, CDCl₃) δ 5.15 (br, 1H), 4.06-4.01 (m, 3H), 3.99-3.95 (m, 1H), 3.93-3.88 (m, 1H), 3.43-3.37 (m, 6H), 1.57-1.53 (m, 2H), 1.503 (s, 9H, the other diastereomer), 1.502 (s, 9H, one diastereomer), 1.25 (m, 30H), 0.89 (s, 9H), 0.88 (t, J = 6.8 Hz, 3H), 0.10 (s, 3H, the other diastereomer), 0.094 (s, 3H, one diastereomer), 0.089 (s, 9H).

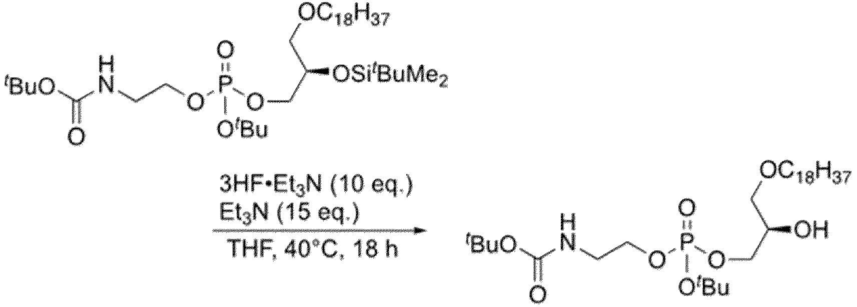

To a solution of *tert*-butyl(2-((*tert*-butoxy((*R*)-2-((*tert*butyldimethylsilyl)oxy)-3-(octadecyloxy)propoxy)phosphoryl)oxy)ethyl)carbamate (980 mg, 1.33 mmol) and triethylamine (2.7 mL, 19.5 mmol) in tetrahydrofuran (13 mL), triethylamine trihydrofluoride (2.2 mL, 13 mmol) was added at room temperature, and the resulting mixture was heated to 40°C, followed by stirring the mixture for 18 hours. Saturated aqueous sodium hydrogen carbonate solution was added to the mixture, and then extraction with chloroform was carried out twice. The chloroform layers obtained by the extraction were combined. The resulting combined layer was washed with water and saturated brine, and dried over Na₂SO₄, followed by removal of the solvent by distillation under reduced pressure. The residue was purified by column chromatography, to obtain a desired product, *tert*-butyl(2-((*tert*-butoxy((R)-2-hydroxy-3-(octadecyloxy)propoxy)phosphoryl)oxy)ethyl)carbamate, as a colorless liquid (1.25 g, 94%).

¹H NMR (500 MHz, CDCl₃) δ 5.17 (br, 1H), 4.14-3.97 (m, 5H), 3.48 (d, J = 5.3 Hz, 2H), 3.45 (t, J = 6.7 Hz, 2H), 3.41 (d, J = 3.41 Hz, 2H), 1.59-1.53 (m, 2H), 1.51 (s, 9H), 1.44 (s, 9H), 1.25 (m, 30H), 0.88 (t, J = 6.9 Hz, 3H).

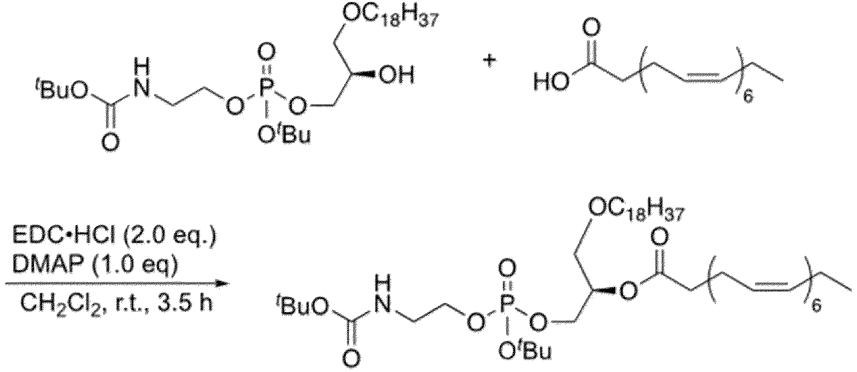

To a solution of *tert*-butyl (2-((*tert*-butoxy((*R*)*-*2-hydroxy-3-(octadecyloxy)propoxy)phosphoryl)oxy)ethyl)carbamate (173 mg, 0.28 mmol) in dichloromethane (6 mL), 1-(3-dimethylaminopropyl)-3-ethylcarbodiimide hydrochloride (106 mg, 0.56 mmol), dimethylaminopyridine (34 mg, 0.28 mmol), and docosahexaenoic acid (92 mg, 0.28 mmol) were added at room temperature, and the resulting mixture was stirred for 3.5 hours. The reaction solution was diluted with ethyl acetate, and the resulting dilution was washed with water. The ethyl acetate layer was dried over anhydrous sodium sulfate, and then concentrated under reduced pressure. The residue was purified by column chromatography, to obtain a desired product, (2*R*)-1-((*tert-*butoxy(2-((tert-butoxycarbonyl)amino)ethoxy)phosphoryl)oxy)-3-(octadecyloxy)propan-2-yl(4*Z*,7*Z*,10*Z*,13*Z*,16*Z*,19*Z*)-docosa-4,7,10,13,16,19-hexaenoate, as a colorless liquid (234 mg, 89%).

¹H NMR (400 MHz, CDCl₃) δ 5.40-5.28 (m, 12H), 5.18-5.13 (m, 2H), 4.21-4.10 (m, 2H), 4.09-4.05 (m, 2H), 3.55 (d, J = 6.6 Hz, 2H), 3.47-3.40 (m, 4H), 2.86-2.80 (m, 10H), 2.41-2.40 (m, 4H), 1.55-1.52 (m, 2H), 1.502 (s, 9H, one diastereomer), 1.496 (s, 9H, the other diastereomer), 1.44 (s, 9H), 1.25 (m, 30H), 0.97 (t, J = 9.4 Hz, 3H), 0.88 (t, J = 8.5 Hz, 3H).

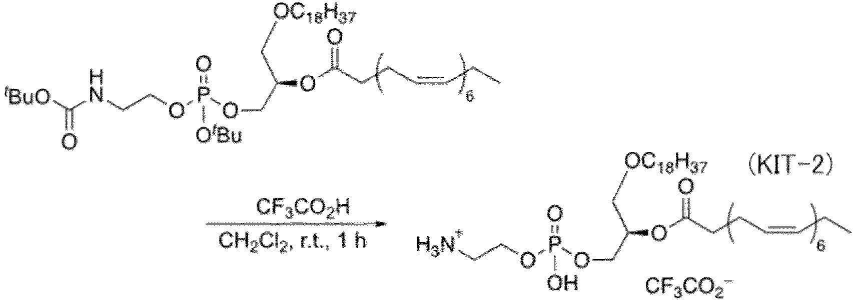

To a solution of (2*R*)-1-((*tert*-butoxy(2-((*tert-*butoxycarbonyl)amino)ethoxy)phosphoryl)oxy)-3-(octadecyloxy)propan-2-yl(4*Z*,7*Z*,10*Z*,13*Z*,16*Z*,19*Z*)-docosa-4,7,10,13,16,19-hexaenoate (182 mg, 0.2 mmol) in dichloromethane (2 mL), 2 mL of trifluoroacetic acid was added at room temperature, and the resulting mixture was stirred for 1 hour. The reaction mixture was concentrated under reduced pressure, to obtain a desired product, 2-((((R)-2-(((4*Z*,7*Z*,10*Z*,13*Z*,16*Z*,19*Z*)-docosa-4,7,10,13,16,19-hexaenoyl)oxy)-3-(octadecyloxy)propoxy)(hydroxy)phosphoryl)oxy)ethane-1-ammonium 2,2,2-trifluoroacetate (KIT-2), as a colorless liquid (167 mg, 94%).

¹H NMR (500 MHz, CDCl₃) δ 8.14 (br, 3H), 5.41-5.28 (m, 12H), 5.16 (br, 1H), 4.10-3.90 (m, 4H), 3.51-3.39 (m, 4H), 3.18 (br, 2H), 2.83-2.81 (m, 10H), 2.38 (m, 4H), 2.07 (t, J = 7.3 Hz, 2H), 1.51 (br, 2H), 1.24 (m, 30H), 0.97 (t, J = 7.5 Hz, 3H), 0.87 (t, J = 0.88 Hz, 3H).

### Test Example 1: Evaluation of Anti-Inflammatory Effect of Compound KIT-13

Under conditions where KIT-2 or KIT-13 was present (5 µg/mL), or where neither KIT-2 nor KIT-13 was present, BV2 cells, which are microglial cells in mice, were cultured for 16 hours in DMEM medium supplemented with 2% fetal bovine serum (FBS). Thereafter, treatment with LPS (1 µg/mL) was carried out for 6 hours. Subsequently, nuclear protein was extracted from the cells, and p65 was detected by the Western blotting method. The antibody used for the detection was an anti-p65 NFkB antibody (Ref no. 51-0500, manufactured by Invitrogen).

In addition, 20 µL of the cell culture supernatant after the LPS treatment was subjected to detection of the inflammatory cytokine IL-1β by the ELISA method using a mouse IL-1β ELISA kit (manufactured by R&D Systems; Catalog No. DY401-05).

### (Results)

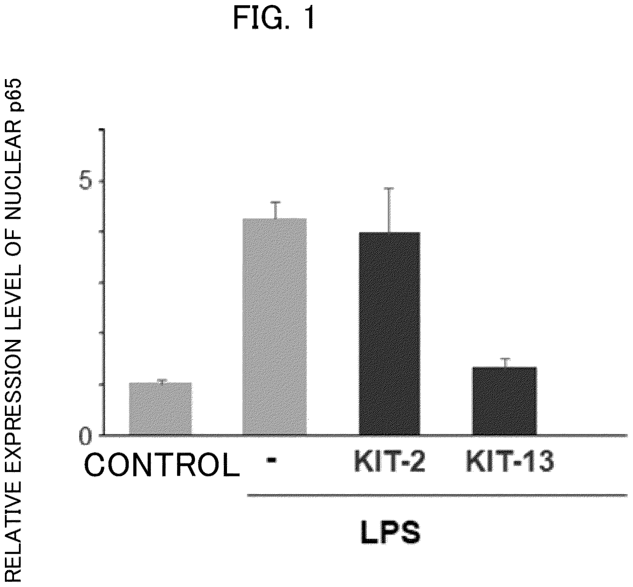

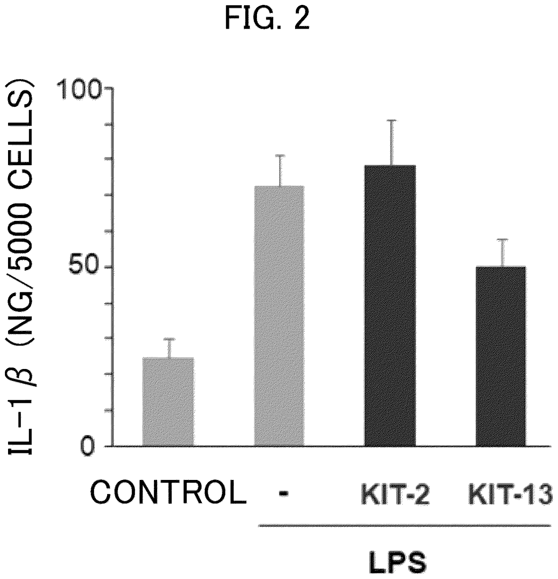

FIG. 1 illustrates the p65 expression level in 20 µg of nuclear protein as compared to an LPS-untreated control (n=2). KIT-13 remarkably suppressed LPS-induced nuclear accumulation of p65. FIG. 2 illustrates the amount of IL-1β protein per 5000 cells (n=4). KIT-13 remarkably decreased LPS-induced expression of the inflammatory cytokine IL-1β.

### Test Example 2: Evaluation of Anti-Inflammatory Effect of Compound KIT-13

Plasmalogen (sPls) was prepared from a scallop (*Mizuhopecten yessoensis*) by the following method. Kokulase P (Mitsubishi-Chemical Foods Corporation) was added to, and mixed with, fresh scallop mantle. Ethanol was added to the resulting mixture, and the mixture was subjected to suction filtration. The resulting filtrate was dried using a rotary evaporator, to obtain sPls.

Under conditions where sPls or KIT-13 was present (5 µg/mL), or where neither sPls nor KIT-13 was present, BV2 cells were cultured for 16 hours in DMEM medium supplemented with 2% fetal bovine serum (FBS). Thereafter, treatment with LPS (1 µg/mL) was carried out for 2 hours. Subsequently, cellular RNA was extracted using the TRIzol (trademark) reagent, and cDNA was prepared from the RNA using a PrimeScript (trademark) 1st strand cDNA Synthesis Kit (manufactured by Takara; 6110). Using TaKaRa Taq (trademark) (manufactured by Takara; R110A), the expression levels of the IL-1β gene and, as an internal standard, the β-actin gene were quantified by polymerase chain reaction (PCR).

The base sequences of the forward primer and the reverse primer for the IL-1β gene are shown in SEQ ID NOs: 1 and 2, respectively. The base sequences of the forward primer and the reverse primer for the β-actin gene are shown in SEQ ID NOs: 3 and 4, respectively.

### (Results)

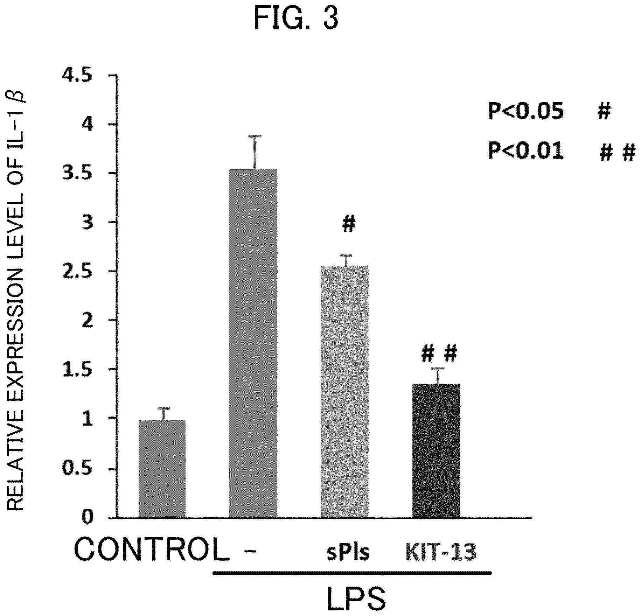

FIG. 3 illustrates the expression level of the IL-1β gene as compared to a control not treated with LPS (the average for three PCR samples prepared from the same cDNA sample). KIT-13 and sPls significantly suppressed the expression of the IL-1β gene as compared to the cases without the addition of KIT-13 or sPls.

### Test Example 3: Evaluation of Lifetime Using RTT Model Mice

In the present Test Example, the effect of KIT-13 on the growth of mice was evaluated using MeCP2-deficient mice, which exhibit a phenotype similar to RTT.

For the test, 4-week-old male MeCP2-deficient mice were used. The MeCP2-deficient mice were divided into the following two groups: a KIT-13 administration group (KO-KIT-13, n = 14) and a control group (KO, n = 28). The body weight of each mouse was measured in advance. For the KIT-13 administration group, KIT-13 was suspended in water by ultrasonication, and administered twice a week by drinking-water administration such that 20 ng/g body weight of KIT-13 was taken. On the other hand, in the control group, only water was given in the same manner.

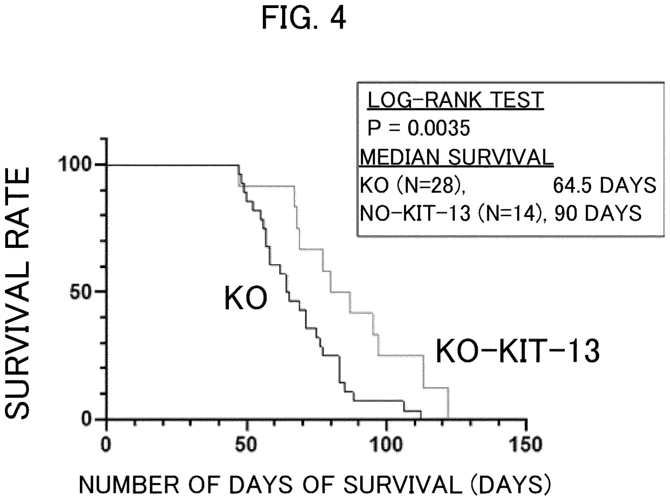

FIG. 4 illustrates the number of days of survival of the mice. The KIT-13 administration group showed a higher survival rate than the control group. The number of days required for the survival rate to decrease to 50% was 64.5 days in the mice in the control group, but the number of days of survival was 90 days in the mice in the KIT-13 administration group. Thus, the RTT model mice to which KIT-13 was administered were shown to have longer lifetimes.

### Test Example 4: Evaluation of RTT-like Scoring Using RTT Model Mice

RTT-like symptoms in MeCP2-deficient mice to which KIT-13 was administered were evaluated by RTT-like scoring. For the test, 4-week-old male MeCP2-deficient mice were used. The MeCP2-deficient mice were divided into the following two groups: a KIT-13 administration group (KO-KIT-13, n = 14) and a control group (KO, n = 15). In the KIT-13 administration group, KIT-13 was administered at 20 ng/g body weight by drinking-water administration in the same manner as in Test Example 3. In the control group, only water was given in the same manner.

The RTT-like scoring was carried out once a week from 5 weeks old, for the following items 1 to 6.

### 1. Mobility

Each mouse was observed after gently placing the mouse on a platform.
0 points: Same as the wild type.
1 point: Lower mobility compared to the wild type. (The period of freezing after the first placement on the platform is longer, or the period during which the mouse is standing still is longer.)
2 points: No spontaneous movement after the placement on the platform. The mouse can move in response to mild stimuli or food placed nearby.

### 2. Gait

0 points: Same as the wild type.
1 point: Due to a severe reduction in the pelvis, the mouse spreads the hindlimbs apart during walking or running, compared to the wild type. Waddling gait. Staggering gait.
2 points: More remarkable abnormality. The mouse swings upon lifting a limb, walks backward, or lifts the hind limbs at the same time.

### 3. Hindlimb Clasping

Each mouse was observed after suspending the mouse by holding the tail base.
0 points: The mouse spreads the hindlimbs outward.
1 point: The mouse pulls a hindlimb toward the other, or pulls one hindlimb toward the body.
2 points: The mouse tightly pulls both hindlimbs. Both hindlimbs attach to each other, or contact the body.

### 4. Tremor

A mouse standing on a flat palm was observed.
0 points: No swinging.
1 point: Intermittent and slight swinging.
2 points: Continuous swinging, or intermittent and severe swinging.

### 5. Breathing

The movement of the flank was observed when the animal was in a resting state.
0 points: Normal breathing.
1 point: Periods with regular breathing and short periods with faster breathing or respiratory arrest sporadically occur.
2 points: Very irregular breathing. Gasping or shortness of breath.

### 6. General condition

Observation was carried out based on general indices of well-being, such as the coat condition (hair condition), eyes, and posture.
0 points: Shiny hair with a good appearance, eyes with a good appearance, and a normal posture.
1 point: Glassy eyes, dull hair/lack of grooming, a slightly round-shouldered posture.
2 points: Eyes kept closed, or narrowed eyes; piloerection; and a round-shouldered posture.

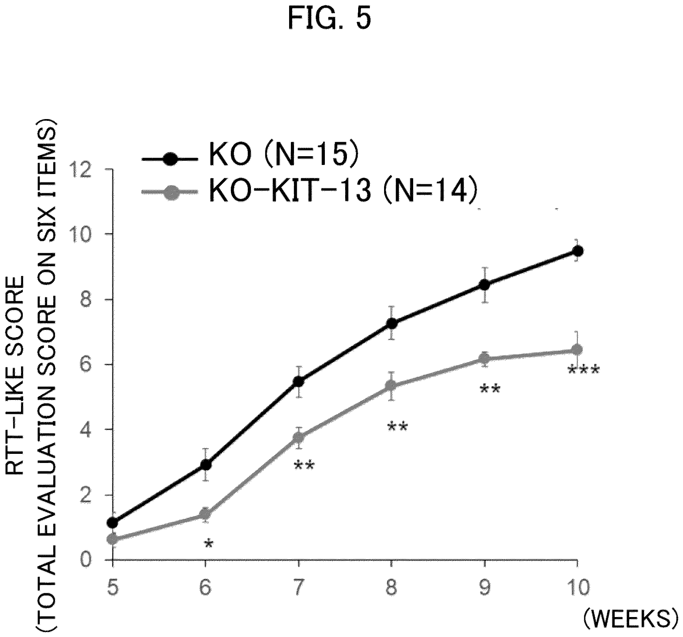

FIG. 5 illustrates changes in the RTT-like score over time. The lower the RTT-like score, the more suppressed the RTT-like symptoms. The administration of KIT-13 reduced the RTT-like score of the mice. Thus, administration of KIT-13 was found to improve the RTT-like symptoms.

### Test Example 5: Evaluation of Body Weight

MeCP2-deficient mice to which KIT-13 was administered were subjected to measurement of the body weight every week, and changes in the body weight were evaluated. For the test, 4-week-old male MeCP2-deficient mice were used. The MeCP2-deficient mice were divided into the following two groups: a KIT-13 administration group (KO-KIT-13, n = 14) and a control group (KO, n = 21). In the KIT-13 administration group, KIT-13 was administered at 20 ng/g body weight by drinking-water administration in the same manner as in Test Example 3. In the control group, only water was given in the same manner.

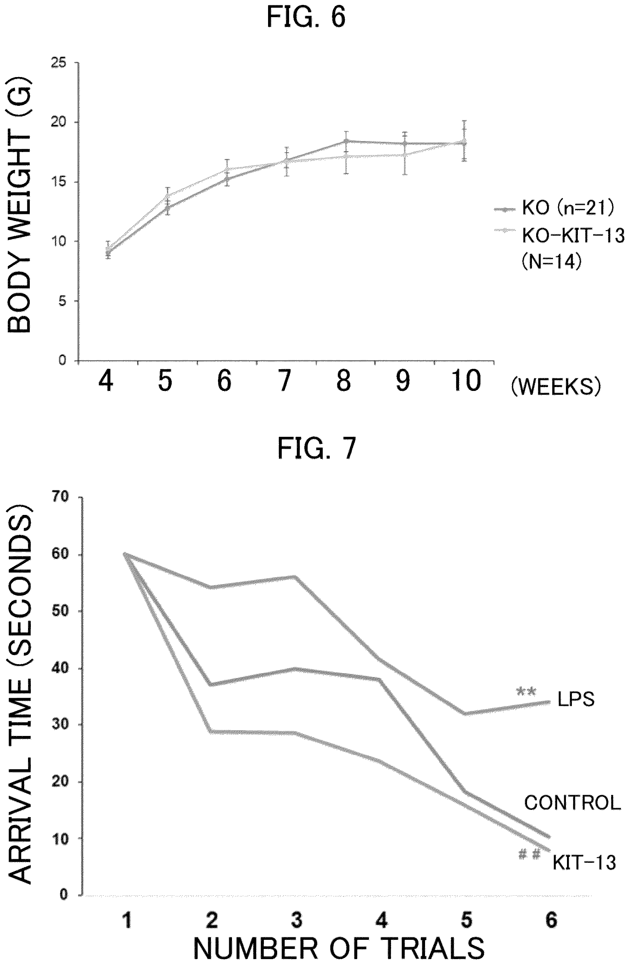

FIG. 6 illustrates changes in the body weight over time. No statistical difference was found between the changes in the body weight in the KIT-13 administration group and the changes in the body weight in the control group (t-test). Thus, KIT-13 is thought to be non-toxic.

### Test Example 6: Evaluation Using LPS-Induced Memory Impairment Model Mice

The effect of KIT-13 on LPS-induced memory impairment in mice was studied. Eight-week-old male c57BL6J mice (5 individuals per group; n = 5) were allowed to drink KIT-13 for 30 days at a dose of 10 mg/50 kg/day, and then LPS treatment (250 mg/kg/day) was carried out for 7 days while the mice were similarly allowed to drink KIT-13. Thereafter, a water maze test was carried out for 2 days (three trials per day).

The time required for the mice to reach an escape platform (arrival time) was as illustrated in FIG. 7. Intergroup comparison was carried out using the ANOVA test and the Bonferroni post-hoc test. The LPS group showed a decrease in the memory (an increase in the arrival time) compared to the control group (P < 0.01). In the sixth trial, the KIT-13 group showed improvement in the memory compared to the LPS group (P < 0.01). Thus, KIT-13 is effective for improvement of the brain function (cognitive function).

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

This application claims the benefit of Japanese Patent Application No. 2021-189168, filed on November 22, 2021, the entire disclosure of which is incorporated by reference herein.

### Industrial Applicability

The present disclosure is useful for pharmaceutical and oral compositions.

## Claims

1. A compound of Formula (I), a racemate of the compound, or a salt of the compound or the racemate, 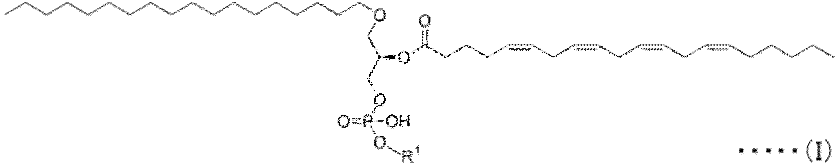 in which each carbon atom of a glycerol backbone optionally has one or more substituents, and R¹ is 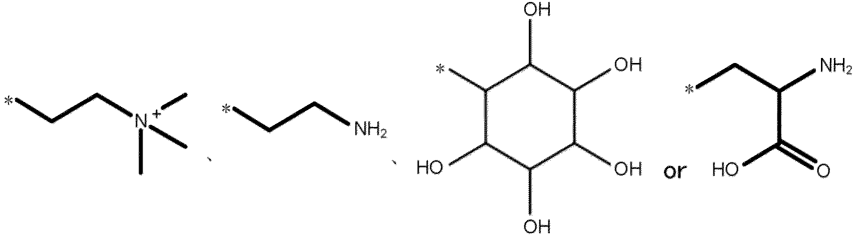 optionally having one or more substituents, in which * is an oxygen atom bound to R¹.

2. A composition comprising the compound, the racemate of the compound, or the salt of the compound or the racemate according to claim 1, as an effective component.

3. An anti-inflammatory agent comprising the composition according to claim 2.

4. The anti-inflammatory agent according to claim 3, wherein the anti-inflammatory agent is for prevention or improvement of a cranial nerve inflammatory disease.

5. A dementia therapeutic agent comprising the composition according to claim 2.

6. A Rett syndrome therapeutic agent comprising the composition according to claim 2.
